# EUROPEAN PATENT APPLICATION

(11) **EP 0 747 024 A2**
(43) Date of publication of application: **11.12.1996**
(21) Application number: 96201608.5
(22) Date of filing: 07.06.1996
(51) Int. Cl.: A61F 2/34, A61L 27/00

(54) **Orthopaedic bearing component and method for making the same**

(30) Priority: 09.06.1995 US 489095
(71) Applicant: Bristol-Myers Squibb Company, New York, N.Y. 10154 (US)
(72) Inventor: Swarts, Dale F., Warsaw, IN 46580 (US); Krebs, Steven L., Fort Wayne, IN 46804 (US); Devanathan, Thirumalai, Warsaw, IN 46580 (US)
(74) Representative: Mays, Julie

(57) **Abstract**

The invention is directed to a method of making a bearing component for use in an orthopaedic implant. A mold is provided which has an inner cavity. A contoured surface is also provided within the mold which has a shape corresponding to a predetermined shape of a bearing surface on the bearing component. A plastic material is introduced into the mold. The plastic material is caused to conform to the contoured surface and thereby define the bearing surface of the bearing component. The plastic material is then removed from the mold, and a portion of the plastic material which is disposed away from the bearing surface is machined to form a load distribution surface.

## Description

### BACKGROUND OF THE INVENTION

### 1. Field of the invention.

The present invention relates to orthopaedic implants, and, more particularly, to orthopaedic implants having a bearing surface formed from a plastic material.

### 2. Description of the related art.

It is known to provide a bearing component for use in an orthopaedic implant which is made from plastic. For example, an implant in the form of an acetabular cup may include a polymeric bearing component having an articulating surface for receiving a femoral head and a load distribution surface for transferring loads to the pelvic bone. The polymeric cup may be attached to a metal backing at the load distribution surface, as is known. The polymeric material may be, e.g., high-density polyethylene (HDPE) or ultra-high molecular weight polyethylene (UHMWPE).

It is known to form the bearing surface and load distribution surface on a polymeric cup by the process of machining. The surface finish for a machined surface of a polymeric cup is typically about 35 to 45 micro-inch (R_{A} = 35-45 µ-in.). Such a surface finish is adequate for the load distribution surface which is attached either to a metal backing or the bone, but may not be smooth enough at the articulating surface for particular applications. Machining has the advantage of being a mass production method with associated cost savings, but may not provide a suitable surface finish on a bearing surface for certain applications.

It is also known to form an entire bearing component by a molding process. For example, polymeric cups can be formed in individual molds, whereby both the articulating surface and the load distribution surface are formed by the process of molding. Molding has the advantage of producing and an articulating surface with a surface finish between 13-22 micro-inch (R_{A} = 13-22 µ-in.). A conventional process for molding polymeric cups is not adapted to mass production, since each polymeric cup must be separately molded. Further, the cost associated with separately molded polymeric cups is relatively high, as compared to machining.

What is needed in the art is a method of manufacturing a bearing component for use in an orthopaedic implant, which combines the advantages of both machining and molding.

### SUMMARY OF THE INVENTION

The present invention provides a method of manufacturing a bearing component for use in an orthopaedic implant, wherein a bearing surface is molded and a load distribution surface is machined.

The invention comprises, in one form thereof, a method of making a bearing component for use in an orthopaedic implant. A mold is provided which has an inner cavity. A contoured surface is also provided within the mold which has a shape corresponding to a predetermined shape of a bearing surface on the bearing component. A plastic material is introduced into the mold. The plastic material is caused to conform to the contoured surface and thereby define the bearing surface of the bearing component. The plastic material is then removed from the mold, and a portion of the plastic material which is disposed away from the bearing surface is machined to form a load distribution surface.

An advantage of the present invention is that a bearing component is manufactured by both molding and machining processes, thereby incorporating advantages associated with each such method of manufacture.

### BRIEF DESCRIPTION OF THE DRAWINGS

The above-mentioned and other features and advantages of this invention, and the manner of attaining them, will become more apparent and the invention will be better understood by reference to the following description of embodiments of the invention taken in conjunction with the accompanying drawings, wherein:
Fig. 1 is an exploded, perspective view of an embodiment of a mold used to form a bearing component of the present invention, with an insert shown in relation thereto;
Fig. 2 is a side, sectional view through the mold bottom shown in Fig. 1, with a plurality of inserts shown in relation thereto;
Fig. 3 is a perspective view of an embodiment of a molded sheet of polyethylene, using the mold of the type shown in Figs. 1 and 2;
Fig. 4 is a fragmentary, sectional view taken along line 4-4 in Fig. 3 along with a mill cutter;
Fig. 5 is a side, sectional view of another embodiment of a mold used to form a bearing component of the present invention; and
Fig. 6 is a perspective view of the mold shown in Fig. 5.

Corresponding reference characters indicate corresponding parts throughout the several views. The exemplifications set out herein illustrate one preferred embodiment of the invention, in one form, and such exemplifications are not to be construed as limiting the scope of the invention in any manner.

### DETAILED DESCRIPTION OF THE INVENTION

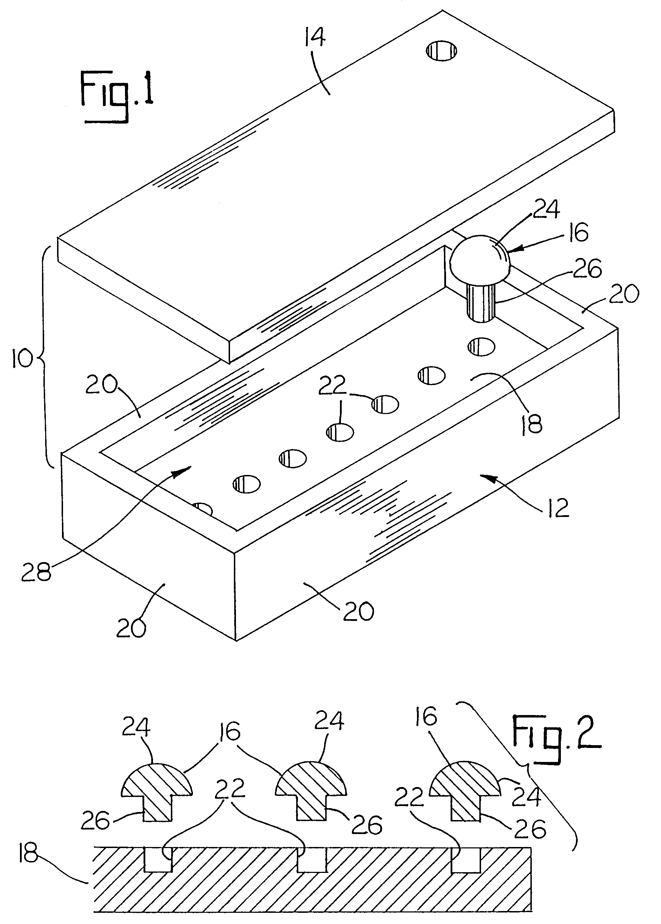

Referring now to the drawings, and more particularly to Figs. 1 and 2, there is shown an embodiment of a mold 10, including a bottom section or drag 12, cover section or cope 14, and a pattern or insert 16.

Drag 12 includes a bottom 18 with a plurality of upstanding walls 20 attached thereto. Bottom 18 has a plurality of recesses 22 for receiving respective inserts 16 therein. Each insert 16 includes a hemispherical portion or contoured surface 24 having a shape which corresponds to a shape of a bearing surface on a bearing component (to be discussed hereinafter). A stem 26 is attached to or integral with contoured surface 24, and is sized to be received within a respective recess 22. When in an installed position within a respective recess 22, each insert 16 is disposed within an inner cavity 28 defined by bottom 18 and walls 20.

Cope 14 is sized and configured to connect with drag 12 after insertion of inserts 16 into recesses 22. An access port 30 allows a plastic material to be introduced into mold 10 after cope 14 is connected to drag 12. Access port 30 may be in the form of an opening in cope 14 as shown, or may be in the form of a nipple, etc.

Fig. 2 is a sectional view taken through three recesses 22 of bottom 18, as shown in relation to three respective inserts 16 (one of which is shown in Fig. 1) prior to assembly therewith.

Referring now to Figs. 3 and 4, a block of plastic material, such as HDPE or UHMWPE, is shown after being removed from a mold similar to mold 10. Plastic block 32 is formed by injecting the plastic material through access port 30 and into inner cavity 28 of mold 10. Plastic block 32 includes a plurality of bearing surfaces 34 which are disposed in a generally square configuration on one side of plastic block 32. Since this configuration is slightly different than the generally linear configuration of recesses 22 formed in bottom 18 of drag 12, it is apparent that inserts 16 may be received within recesses 22 of bottom 18 at a number of predetermined configurations. In the embodiment shown in Figs. 3 and 4, each bearing surface 34 is an articulating surface of an acetabular cup. Bearing surfaces 34 are disposed in plastic block 32 such that plastic block 32 may be separated into a plurality of smaller blocks 36, such as by cutting block 32 along dotted lines 38 and 40. Each smaller block 36 has overall dimensions which are larger than that of a finished acetabular cup.

Referring now to Fig. 4, a sectional view of a smaller block 36 is shown in relation to a milling cutter 42 used to machine a portion of the plastic material of smaller block 36 which is disposed away from bearing surface 34. More particularly, milling cutter 42 and/or smaller block 36 may be moved relative to each other to mill a portion of the plastic material away (as indicated by notch 44) to form a load distribution surface associated with bearing surface 34. In the embodiment shown, the plastic material is machined from smaller block 36, such as by milling, to define a load distribution surface of an acetabular cup which is disposed radially outward from and generally parallel to bearing surface 34. After machining smaller block 36, molded bearing surface 34 has a surface finish between 13 to 22 micro-inch, and the load distribution surface has a surface finish at any particular point between 35 to 45 micro-inch.

In use, plastic material is introduced into inner cavity 28 of mold 10 through access port 30. The mold is provided with an insert 16 having a contoured surface 22 therein with a shape corresponding to a shape of bearing surface 34 (Figs. 3 and 4). The plastic material is caused to conform to contoured surface 24 and thereby define bearing surface 34. After the molding process is complete, cope 14 is removed from drag 12 and plastic block 32 is removed from mold 10. Plastic block 32 is then cut into smaller blocks 36, and a portion of the plastic material disposed away from bearing surface 34 of each smaller block 36 is machined, such as with milling cutter 42, to form a load distribution surface associated with the respective bearing surface.

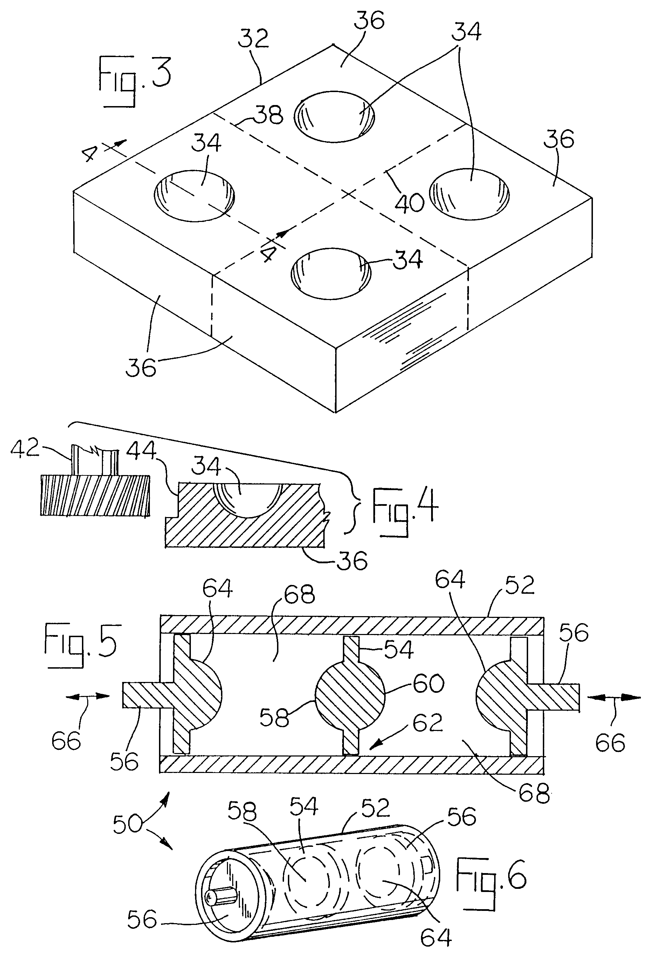

Referring now to Figs. 5 and 6, another embodiment of a mold 50 of the present invention is shown. Mold 50 includes a cylindrical casing 52, in which is disposed at least one slidable pattern or insert 54. A pair of pistons 56 close each respective end of cylindrical casing 52.

Each slidable insert 54, one of which is shown in Figs. 5 and 6, is disposed radially inside of casing 52, and includes a pair of hemispherical portions or contoured surfaces 58 and 60 having a shape corresponding to a bearing surface on a bearing component, such as bearing surface 34 shown in Figs. 3 and 4. Insert 54 is slidable within casing 52, but includes a circumferential interface surface 62 which is sufficient to prevent canting of insert 54 within casing 52.

Each piston 56 includes a hemispherical portion or contoured surface 64 having a shape corresponding to a shape of a bearing surface, such as bearing surface 34 shown in Figs. 3 and 4. Pistons 56 may be moved in either axial direction within casing 52, as indicated by directional arrows 66. Inner cavities 68 are formed between insert 54 and a piston 56, or may be formed between two inserts 54 if more than one insert 54 is utilized.

In use, a piston 56 is placed within casing 52 and plastic material is introduced into casing 62 against the piston 56. An insert 54 is then slid within casing 52 and against the plastic material. Plastic material is then placed on the opposite side of insert 54, and either another insert 54 or the other piston 56 is slid within casing 52. It is thus possible to have a number of inner cavities 68 within mold 50 in which the plastic material is disposed. The number of inner cavities depends upon the number of inserts 54 placed within casing 52. One or both of pistons 56 are then moved in an axial direction within casing 52 to compress the plastic material. The compressive force of pistons 56 on the plastic material disposed within each inner cavity 68 compresses the plastic material and causes the plastic material to conform to a shape of contoured surfaces 58, 60, 64, and thereby define respective bearing surfaces of a bearing component. Casing 52 may also be adapted to include a heating element to heat the plastic material disposed therein. After molding the plastic material within mold 50, pistons 56 and inserts 54 are removed from within casing 52, yielding blocks of plastic material having a molded bearing surface therein. Each block of plastic material can be separated into two halves to form smaller blocks of plastic material, with each smaller block having overall dimensions which are larger than that of a finished acetabular cup. A surface disposed away from the bearing surface is then machined, resulting in the formation of a load distribution surface which is associated with the respective bearing surface. The bearing surface is thus formed by molding; and the load distribution surface is thus formed by machining.

While this invention has been described as having a preferred design, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A method of making a bearing component for use in an orthopaedic implant, comprising the steps of:
providing a mold having an inner cavity;
providing a contoured surface within said mold having a shape corresponding to a predetermined shape of a bearing surface on said bearing component;
introducing a plastic material into said mold;
causing said plastic material to conform to said contoured surface and thereby define said bearing surface of said bearing component;
removing said plastic material from said mold; and
machining a portion of said plastic material which is disposed away from said bearing surface.

2. The method of Claim 1, wherein said step of providing said surface comprises providing a plurality of inserts within said inner cavity.

3. The method of Claim 2, wherein said inserts include a generally hemispherical portion with a stem attached thereto, said mold having a plurality of recesses for respectively receiving said stems.

4. The method of Claim 1, wherein said causing step comprises causing said plastic material to entirely cover said contoured surface.

5. The method of Claim 1, wherein said introducing step comprises injecting said plastic material into said mold under high pressure.

6. The method of Claim 1, wherein said plastic material consists essentially of ultra-high molecular weight polyethylene.

7. The method of Claim 1, wherein said mold comprises a cylindrical casing and a pair of pistons closing each end of said casing.

8. A bearing component for use in an orthopaedic implant, said bearing component including a bearing surface and a load distribution surface, said bearing component manufactured by the process of:
providing a block of plastic material;
molding a plurality of the bearing surfaces in said block; and
machining a plurality of the load distribution surfaces in said block,each said load distribution surface associated with a respective said bearing surface.

9. The bearing component of Claim 8, manufactured by the further step of separating said block into a plurality of smaller blocks, each said smaller block including one of said bearing surfaces.

10. A bearing component for use in an orthopaedic implant, said bearing component comprising:
a bearing surface having a surface finish between a range of approximately 13 to 22 micro-inch; and
a load distribution surface disposed away from said bearing surface and having a surface finish at any particular point between a range of approximately 35 to 45 micro-inch.

11. The bearing component of Claim 10, wherein said bearing component comprises an acetabular cup.

12. The bearing component of Claim 10, wherein said bearing surface is formed by the process of molding, and said load distribution surface is formed by the process of machining.
